# EUROPEAN PATENT SPECIFICATION

(11) **EP 2 266 603 B1**
(45) Date of publication and mention of the grant of the patent: **12.09.2012**
(21) Application number: 10170408.8
(22) Date of filing: 16.10.2001
(51) Int. Cl.: A61K 39/39, A61P 35/00

(54) **Tumour vaccines**
Impfstoffe gegen Krebs
Vaccins contre le cancer

(30) Priority: 18.10.2000 GB 0025573; 18.10.2000 GB 0025574; 18.10.2000 US 690921
(43) Date of publication of application: 29.12.2010
(62) Divisional of application: 07121675.8
(73) Proprietor: GlaxoSmithKline Biologicals S.A., 1330 Rixensart (BE)
(72) Inventor: Garcon, Nathalie, B-1330, Rixensart (BE); Gerard, Catherine Marie Ghislaine, B-1330, Rixensart (BE); Stephenne, Jean, B-1330, Rixensart (BE)
(74) Representative: O'Farrell, Damien John

(56) References cited:
- WO-A-00/09159
- WO-A-00/62800
- WO-A-99/40188
- WO-A-99/61056
- WO-A1-2010/136443
- WO-A2-00/53216
- WO-A2-01/02828
- WO-A2-01/62778
- BALL D W ET AL: "Identification of a human achaete-scute homolog highly expressed in neuroendocrine tumors." PROCEEDINGS OF THE NATIONAL ACADEMY OF SCIENCES OF THE UNITED STATES OF AMERICA 15 JUN 1993 LNKD- PUBMED:8390674, vol. 90, no. 12, 15 June 1993 (1993-06-15) , pages 5648-5652, XP002606807 ISSN: 0027-8424

### SEQUENCE LISTING

<110> GlaxoSmithKline Biologicals s.a.
<120> Vaccines
<130> B45245D2EP
<150> GB20000025573
   <151> 2000-10-18
<150> GB20000025574
   <151> 2000-10-18
<150> US20000690921
   <151> 2000-10-18
<160> 5
<170> PatentIn version 3.5
<210> 1
   <211> 20
   <212> DNA
   <213> Artificial Sequence
<220>
   <223> Immunostimulatory oligonucleotide
<400> 1
   tccatgacgt tcctgacgtt
   20
<210> 2
   <211> 18
   <212> DNA
   <213> Artificial Sequence
<220>
   <223> Immunostimulatory sequence
<400> 2
   tctcccagcg tgcgccat
   18
<210> 3
   <211> 30
   <212> DNA
   <213> Artificial Sequence
<220>
   <223> Immunostimulatory oligonucleotide
<400> 3
   accgatgacg tcgccggtga cggcaccacg
   30
<210> 4
   <211> 24
   <212> DNA
   <213> Artificial Sequence
<220>
   <223> Immunostimulatory oligonucleotide
<400> 4
   tcgtcgtttt gtcgttttgt cgtt
   24
<210> 5
   <211> 20
   <212> DNA
   <213> Artificial Sequence
<220>
   <223> Immunostimulatory oligonucleotide
<400> 5
   tccatgacgt tcctgatgct
   20

## Description

The present invention relates to a novel immunogenic composition comprising a combination of a ASCL1 or ASCL2 cancer antigen and a combined adjuvant composition comprising an immunostimulatory oligonucleotide, a saponin and a lipopolysaccharide, as defined in claim 1.

Despite enormous investments of financial and human resources, cancer remains one of the major causes of death. For example, cancer is the leading cause of death in women between the ages of 35 and 74. Breast cancer is the most common malignancy in women and the incidence for developing breast cancer is on the rise. It is estimated that one in nine women will be diagnosed with the disease. Standard approaches to cure breast cancer have centered around a combination of surgery, radiation and chemotherapy. These approaches have resulted in some dramatic successes in certain malignancies. However, breast cancer is most often incurable, when diagnosed beyond a certain stage. Alternative approaches to early diagnosis and therapy are necessary.

WO 01/62778 published on 30.08.2001 discloses immunogenetic compositions comprising HASH-2 and an adjuvant comprising CpG and saponin or QS21 and 3D-MPL.

Immunostimulatory oligonucleotides containing unmethylated CpG dinucleotides ("CpG") and are known in the art as being adjuvants when administered by both systemic and mucosal routes (WO 96/02555, EP 468520, Davis et al., J.Immunol, 1998, 160(2):870-876; McCluskie and Davis, J.Immunol., 1998, 161 (9):4463-6). CpG is an abbreviation for cytosine-guanosine dinucleotide motifs present in DNA. Historically, it was observed that the DNA fraction of BCG could exert an anti-tumour effect. In further studies, synthetic oligonucleotides derived from BCG gene sequences were shown to be capable of inducing immunostimulatory effects (both in vitro and in vivo). The authors of these studies concluded that certain palindromic sequences, including a central CG motif, carried this activity. The central role of the CG motif in immunostimulation was later elucidated in a publication by Krieg, Nature 374, p546 1995. Detailed analysis has shown that the CG motif has to be in a certain sequence context, and that such sequences are common in bacterial DNA but are rare in vertebrate DNA. The immunostimulatory sequence is often: Purine, Purine, C, G, pyrimidine, pyrimidine; wherein the dinucleotide CG motif is not methylated, but other unmethylated CpG sequences are known to be immunostimulatory and may be used in the present invention.

In certain combinations of the six nucleotides a palindromic sequence is present. Several of these motifs, either as repeats of one motif or a combination of different motifs, can be present in the same oligonucleotide. The presence of one or more of these immunostimulatory sequence containing oligonucleotides can activate various immune subsets, including natural killer cells (which produce interferon y and have cytolytic activity) and macrophages (Wooldrige et al Vol 89 (no. 8), 1977). Although other unmethylated CpG containing sequences not having this consensus sequence have now been shown to be immunomodulatory.

CpG when formulated into vaccines, is generally administered in free solution together with free antigen (WO 96/02555; McCluskie and Davis, *supra*) or covalently conjugated to an antigen (PCT Publication No. WO 98/16247), or formulated with a carrier such as aluminium hydroxide ((Hepatitis surface antigen) Davis *et al. supra* ; Brazolot-Millan et al., Proc.Natl.Acad. Sci. , USA, 1998, 95(26), 15553-8).

The adjuvant combinations of the present invention include at least one enterobacterial lipopolysaccharide derived adjuvant.

It has long been known that enterobacterial lipopolysaccharide (LPS) is a potent stimulator of the immune system, although its use in adjuvants has been curtailed by its toxic effects. A non-toxic derivative of LPS, monophosphoryl lipid A (MPL), produced by removal of the core carbohydrate group and the phosphate from the reducing-end glucosamine, has been described by Ribi et al (1986, Immunology and Immunopharmacology of bacterial endotoxins, Plenum Publ. Corp., NY, p407-419) and has the following structure:

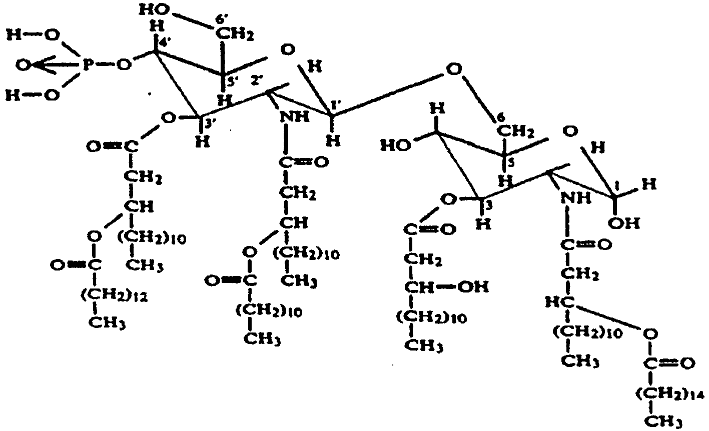

A further detoxified version of MPL results from the removal of the acyl chain from the 3-position of the disaccharide backbone, and is called 3-O-Deacylated monophosphoryl lipid A (3D-MPL). It can be purified and prepared by the methods taught in GB 2122204B, which reference also discloses the preparation of diphosphoryl lipid A, and 3-O-deacylated variants thereof. A preferred form of 3D-MPL is in the form of an emulsion having a small particle size less than 0.2µm in diameter, and its method of manufacture is disclosed in WO 94/21292. Aqueous formulations comprising monophosphoryl lipid A and a surfactant have been described in WO 98/43670A2.

The bacterial lipopolysaccharide derived adjuvants to be formulated in the adjuvant combinations of the present invention may be purified and processed from bacterial sources, or alternatively they may be synthetic. For example, purified monophosphoryl lipid A is described in Ribi et al 1986 (supra), and 3-O-Deacylated monophosphoryl or diphosphoryl lipid A derived from *Salmonella sp.* is described in GB 2220211 and US 4912094. Other purified and synthetic lipopolysaccharides have been described (WO 98/01139; US 6,005,099 and EP 0 729 473 B1; Hilgers et al., 1986, INt.Arch.Allergy. Immunol, 79(4):392-6; Hilgers et al., 1987, Immunology, 60(1): 141-6; and EP 0 549 074 B1). Particularly preferred bacterial lipopolysaccharide adjuvants are 3D-MPL and the β(1-6) glucosamine disaccharides described in US 6,005,099 and EP 0 729 473 B1.

Accordingly, the LPS derivatives that may be used in the present invention are DPL or MPL or 3D-MPL.

A preferred disaccharide adjuvant is a purified or synthetic lipid A of the following formula: 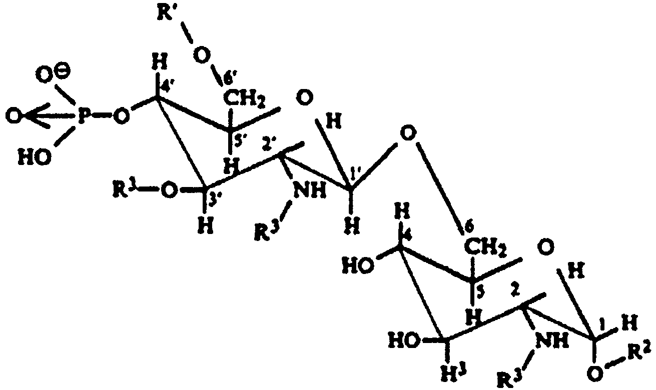 wherein R2 may be H or PO3H2; R3 may be an acyl chain or β-hydroxymyristoyl or a 3-acyloxyacyl residue having the formula: 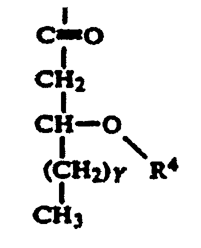 wherein R⁴ = 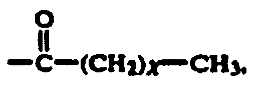 and wherein X and Y have a value of from 0 up to about 20.

Combinations of 3D-MPL and saponin adjuvants derived from the bark of Quillaja Saponaria molina have been described in EP 0 761 231 B. WO 95/17210 discloses an adjuvant emulsion system based on squalene, α-tocopherol, and polyoxyethylene sorbitan monooleate (TWEEN80), formulated with the immunostimulant QS21, optionally with 3D-MPL.

Saponins are known as adjuvants in vaccines for systemic administration. The adjuvant and haemolytic activity of individual saponins has been extensively studied in the art (Lacaille-Dubois and Wagner, *supra*). For example, Quil A (derived from the bark of the South American tree Quillaja Saponaria Molina), and fractions thereof, are described in US 5,057,540 and "Saponins as vaccine adjuvants", Kensil, C. R., Crit Rev Ther Drug Carrier Syst, 1996, 12 (1-2):1-55; and EP 0 362 279 B1.

Particulate structures, termed Immune Stimulating Complexes (ISCOMS), comprising fractions of Quil A are haemolytic and have been used in the manufacture of vaccines (Morein, B., EP 0 109 942 B1). These structures have been reported to have adjuvant activity (EP 0 109 942 B1; WO 96/11711).

The haemolytic saponins QS21 and QS17 (HPLC purified fractions of Quil A) have been described as potent systemic adjuvants, and the method of their production is disclosed in US Patent No.5,057,540 and EP 0 362 279 B1. Also described in these references is the use of QS7 (a non-haemolytic fraction of Quil-A) which acts as a potent adjuvant for systemic vaccines. Use of QS21 is further described in Kensil et al. (1991. J. Immunology vol 146, 431-437). Combinations of QS21 and polysorbate or cyclodextrin are also known (WO 99/10008). Particulate adjuvant systems comprising fractions of QuilA, such as QS21 and QS7 are described in WO 96/33739 and WO 96/11711.

Other saponins which have been used in systemic vaccination studies include those derived from other plant species such as Gypsophila and Saponaria (Bomford et al., Vaccine, 10(9):572-577, 1992).

Saponins are also known to have been used in mucosally applied vaccine studies, which have met with variable success in the induction of immune responses. Quil-A saponin has previously been shown to have no effect on the induction of an immune response when antigen is administered intranasally (Gizurarson et al. 1994. Vaccine Research 3, 23-29). Whilst, other authors have used this adjuvant with success (Maharaj et al., Can.J.Microbiol, 1986, 32(5):414-20; Chavali and Campbell, Immunobiology, 174(3):347-59). ISCOMs comprising Quil A saponin have been used in intragastric and intranasal vaccine formulations and exhibited adjuvant activity (McI Mowat et al., 1991, Immunology, 72, 317-322; McI Mowat and Donachie, Immunology Today, 12, 383-385).

QS21, the non-toxic fraction of Quil A, has also been described as an oral or intranasal adjuvant (Sumino et al., J.Virol., 1998, 72(6):4931-9; WO 98/56415).

Saponins are taught in: Lacaille-Dubois, M and Wagner H. (1996. A review of the biological and pharmacological activities of saponins. Phytomedicine vol 2 pp 363-386). Saponins are steroid or triterpene glycosides widely distributed in the plant and marine animal kingdoms. Saponins are noted for forming colloidal solutions in water which foam on shaking, and for precipitating cholesterol. When saponins are near cell membranes they create pore-like structures in the membrane which cause the membrane to burst. Haemolysis of erythrocytes is an example of this phenomenon, which is a property of certain, but not all, saponins.

The present invention relates to the surprising finding that immunostimulatory oligonucleotides (CpG) and saponin and optionally a lipopolysaccharide combinations are extremely potent adjuvants. Accordingly, there is provided a vaccine combination comprising a combination of saponin and an immunostimulatory oligonucleotide and a lipopolysaccharide with one of the cancer antigens ASCL1 (HASH-1) or ASCL2 (HASH -2) , preferably QS21, an immunostimulatory oligonucleotide, and a 3D-MPL.

Preferably, the vaccine of the present invention may further comprise a carrier. The oligonucleotides in the adjuvant and vaccine compositions may synergistically with the combined saponin/lipolysaccharide in the induction of antigen specific immune responses leading to enhanced tumour regression. The formulations are potent in the induction of immune responses conventionally associated with the Th1-type immune system. Accordingly, the adjuvant combinations are not only suitable for immunoprophylaxis of diseases, but also for immunotherapy of diseases such as cancer.

The formulations contain an anti-tumour antigen and are useful for the immunotherapeutic treatment of cancers.

The preferred oligonucleotides for use in adjuvants or vaccines of the present invention preferably contain two or more dinucleotide CpG motifs separated by at least three, more preferably at least six or more nucleotides. The oligonucleotides of the present invention are typically deoxynucleotides. In a preferred embodiment the internucleotide in the oligonucleotide is phosphorodithioate, or more preferably a phosphorothioate bond, although phosphodiester and other internucleotide bonds are within the scope of the invention including oligonucleotides with mixed internucleotide linkages. Methods for producing phosphorothioate oligonucleotides or phosphorodithioate are described in US5,666,153, US5,278,302 and WO95/26204.

Examples of preferred oligonucleotides have the following sequences. The sequences preferably contain phosphorothioate modified internucleotide linkages. OLIGO 1 (SEQ ID NO:1): TCC ATG ACG TTC CTG ACG TT (CpG 1826) OLIGO 2 (SEQ ID NO:2): TCT CCC AGC GTG CGC CAT (CpG 1758) OLIGO 3(SEQ ID NO:3): ACC GAT GAC GTC GCC GGT GAC GGC ACC ACG OLIGO 4 (SEQ ID NO:4): TCG TCG TTT TGT CGT TTT GTC GTT (CpG 2006) OLIGO 5 (SEQ ID NO:5): TCC ATG ACG TTC CTG ATG CT (CpG 1668)

Alternative CpG oligonucleotides may comprise the preferred sequences above in that they have inconsequential deletions or additions thereto.

The CpG oligonucleotides utilised in the present invention may be synthesized by any method known in the art (eg EP 468520). Conveniently, such oligonucleotides may be synthesized utilising an automated synthesizer. They are typically between 10-50 bases in length.

The oligonucleotides utilised in the present invention are typically deoxynucleotides. In a preferred embodiment the internucleotide bond in the oligonucleotide is phosphorodithioate, or more preferably phosphorothioate bond, although phosphodiesters are within the scope of the present invention. Oligonucleotide comprising different internucleotide linkages are contemplated, e.g. mixed phosphorothioate phophodiesters. Other internucleotide bonds which stabilise the oligonucleotide may be used.

The saponins which may be used in the adjuvant combinations of the present invention include those derived from the bark of Quillaja Saponaria Molina, termed Quil A, and fractions thereof, described in US 5,057,540 and "Saponins as vaccine adjuvants", Kensil, C. R., Crit Rev Ther Drug Carrier Syst, 1996, 12 (1-2):1-55; and EP 0 362 279 B1. Particularly preferred fractions of Quil A are QS21, QS7, and QS17.

β-Escin is another preferred haemolytic saponins for use in the adjuvant compositions of the present invention. Escin is described in the Merck index (12^{th} ed: entry 3737) as a mixture of saponins occurring in the seed of the horse chestnut tree, Lat: *Aesculus hippocastanum.* Its isolation is described by chromatography and purification (Fiedler, Arzneimittel-Forsch. 4, 213 (1953)), and by ion-exchange resins (Erbring *et al.,* US 3,238,190). Fractions of escin, □and □, have been purified and shown to be biologically active (Yoshikawa M, et al. (Chem Pharm Bull (Tokyo) 1996 Aug;44(8):1454-1464)). β-escin is also known as aescin.

Another preferred haemolytic saponin for use in the present invention is Digitonin. Digitonin is described in the Merck index (12th Edition, entry 3204) as a saponin, being derived from the seeds of *Digitalis purpurea* and purified according to the procedure described Gisvold et al., J.Am.Pharm.Assoc., 1934, 23, 664; and Ruhenstroth-Bauer, Physiol.Chem., 1955, 301, 621. Its use is described as being a clinical reagent for cholesterol determination.

The adjuvant combinations may further comprise a carrier, such that the saponin or CpG, or lipolysaccaharide may be associated with a particulate carrier entity to enhance the adjuvanticity of the combination. Particularly preferred systemic vaccines, for example, comprise a carrier molecule.

The CpG used in the adjuvant combinations may be in free solution or may be complexed to particulate carriers such as mineral salts (for example, but not restricted to, aluminium or calcium salts), liposomes, ISCOMs, emulsions (oil in water, water in oil, water in oil in water), polymers (such as, but not restricted to polylactic, polyglycolic, polyphosphazine, polyaminoacid, alginate, chitosan) or microparticles. Preferably said carriers are cationic. The vaccines of the present invention further comprise an antigen which may be associated with the CpG-carrier complex, or may not be associated with the CpG-carrier complex. In this case, the antigen may be free suspension or associated with a separate carrier.

The saponins may be separate in the form of micelles, or may be in the form of large ordered structures such as ISCOMs (EP 0 109 942 B1) or liposomes (WO 96/33739) when formulated with cholesterol and lipid, or in the form of an oil in water emulsion (WO 95/17210). The saponins may preferably be associated with a metallic salt, such as aluminium hydroxide or aluminium phosphate (WO 98/15287). Alternatively the saponin may be associated with a particulate carrier such as chitosan. The saponin may also be in a dry state such as a powder. The final formulations in the form as they are administered to the mucosal surface of the vaccinee are preferably haemolytic in nature. The saponin may or may not be associated physically with the antigen either through direct linkage or by co-interaction with the same particulate carrier molecule (GB9822712.7; WO 98/16247).

The CpG and saponin and lipopolysaccharide in the vaccines of the present invention may themselves be separate or associated. For example, the CpG and saponin may be in free suspension or may be associated via a carrier, more preferably a particulate carrier such as aluminium hydroxide or by a cationic liposome or ISCOM.

A preferred adjuvant combination is composed of one or more CpG oligonucleotides containing at least 3, preferably at least 6 nucleotides between two adjacent CG motifs, together with QS21 and 3D-MPL and a particulate carrier selected from the group comprising an oil-in-water emulsion or DQ. The lipopolysacchharide is a di or monophosphoryl lipid derivative, preferably 3 de-O acylated, in particular 3 de O acylated monophosphoryl Lipid A. Most preferably, the adjuvant combination comprises CpG 2006 (SEQ ID NO: 4), or CpG 1758 (SEQ ID NO: 2) or CpG 1826 (SEQ ID NO: 1) mixed with QS21. Accordingly, particularly preferred vaccines, for example, comprise such adjuvant combinations and an antigen. The preferred vaccine of the present invention is used to generate systemic immune responses after administration to an individual through the systemic route.

The adjuvant combinations can comprise an oil based emulsion. Oil emulsion adjuvants have been known for many years, including work on Freunds complete and incomplete mineral oil emulsion adjuvants. Since that time much work has been performed to design stable and well tolerated alternatives to these potent, but reactogenic, adjuvant formulations.

Many single or multiphase emulsion systems have been described. Oil in water emulsion adjuvants per se have been suggested to be useful as adjuvant compositions (EP O 399 843B), also combinations of oil in water emulsions and other active agents have been described as adjuvants for vaccines (WO 95/17210; WO 98/56414; WO 99/12565; WO 99/11241). Other oil emulsion adjuvants have been described, such as water in oil emulsions (US 5,422,109; EP 0 480 982 B2) and water in oil in water emulsions (US 5,424,067; EP 0 480 981 B).

The oil emulsion adjuvants may be natural or synthetic, and may be mineral or organic. Examples of mineral and organic oils will be readily apparent to the man skilled in the art.

In order for any oil in water composition to be suitable for human administration, the oil phase of the emulsion system preferably comprises a metabolisable oil. The meaning of the term metabolisable oil is well known in the art. Metabolisable can be defined as "being capable of being transformed by metabolism" (Dorland's Illustrated Medical Dictionary, W.B. Sanders Company, 25th edition (1974)). The oil may be any vegetable oil, fish oil, animal oil or synthetic oil, which is not toxic to the recipient and is capable of being transformed by metabolism. Nuts (such as peanut oil), seeds, and grains are common sources of vegetable oils. Synthetic oils are also part of this invention and can include commercially available oils such as NEOBEE® and others. Squalene (2,6,10,15,19,23-Hexamethyl-2,6,10,14,18,22-tetracosahexaene) is an unsaturated oil which is found in large quantities in shark-liver oil, and in lower quantities in olive oil, wheat germ oil, rice bran oil, and yeast, and is a particularly preferred oil for use in this invention. Squalene is a metabolisable oil virtue of the fact that it is an intermediate in the biosynthesis of cholesterol (Merck index, 10th Edition, entry no.8619).

Particularly preferred oil emulsions are oil in water emulsions, and in particular squalene in water emulsions.

In addition, the most preferred oil emulsion adjuvants comprise an antioxidant, which is preferably the oil α-tocopherol (vitamin E, EP 0 382 271 B1).

WO 95/17210 and WO 99/11241 disclose emulsion adjuvants based on squalene, α-tocopherol, and TWEEN 80, optionally formulated with the immunostimulants QS21 and/or 3D-MPL. WO 99/12565 discloses an improvement to these squalene emulsions with the addition of a sterol into the oil phase. Additionally, a triglyceride, such as tricaprylin (C27H50O6 may be added to the oil phase in order to stabilise the emulsion (WO 98/56414).

The size of the oil droplets found within the stable oil in water emulsion are preferably less than 1 micron, may be in the range of substantially 30-600nm, preferably substantially around 30-500nm in diameter, and most preferably substantially 150-500nm in diameter, and in particular about 150 nm in diameter as measured by photon correlation spectroscopy. In this regard, 80% of the oil droplets by number should be within the preferred ranges, more preferably more than 90% and most preferably more than 95% of the oil droplets by number are within the defined size ranges. The amounts of the components present in the oil emulsions are conventionally in the range of from 2 to 10% oil, such as squalene; and when present, from 2 to 10% alpha tocopherol; and from 0.3 to 3% surfactant, such as polyoxyethylene sorbitan monooleate. Preferably the ratio of oil: alpha tocopherol is equal or less than 1 as this provides a more stable emulsion. Span 85 may also be present at a level of about I %. In some cases it may be advantageous that the vaccines of the present invention will further contain a stabiliser.

The method of producing oil in water emulsions is well known to the man skilled in the art. Commonly, the method comprises the mixing the oil phase with a surfactant such as a PBS/TWEEN80™ solution, followed by homogenisation using a homogenizer, it would be clear to a man skilled in the art that a method comprising passing the mixture twice through a syringe needle would be suitable for homogenising small volumes of liquid. Equally, the emulsification process in microfluidiser (M110S microfluidics machine, maximum of 50 passes, for a period of 2 minutes at maximum pressure imput of 6 bar (output pressure of about 850 bar)) could be adapted by the man skilled in the art to produce smaller or larger volumes of emulsion. This adaptation could be achieved by routine experimentation comprising the measurement of the resultant emulsion until a preparation was achieved with oil droplets of the required diameter.

The adjuvant combinations may be used as both systemic or mucosal adjuvant. In a particular form of the invention there is provided a systemic vaccine to be administered through the systemic or parenteral route such as intramuscular, intradermal, transdermal, subcutaneous, intraperitoneal or intravenous administration. A preferred route of administration is via the transdermal route, for example by skin patches.

The systemic vaccine preparations of the present invention may be used to protect or treat a mammal susceptible to, or suffering from disease, by means of administering said vaccine by intramuscular, intraperitoneal, intradermal, transdermal, intravenous, or subcutaneous administration. Methods of systemic administration of the vaccine preparations may include conventional syringes and needles, or devices designed for ballistic delivery of solid vaccines (WO 99/27961), or needleless pressure liquid jet device (US 4,596,556; US 5,993,412), or transdermal patches (WO 97/48440; WO 98/28037), and may also be used to enhance the immunogenicity of antigens applied to the skin (transdermal or transcutaneous delivery WO 98/20734 ; WO 98/28037). A delivery device for systemic administration, pre-filled with the vaccine compositions of the present invention Accordingly there is provided a method for inducing an immune response in an individual, comprising the administration of a vaccine is disclosed, as defined in the claims , to the individual, wherein the vaccine is administered via the parenteral or systemic route.

Alternatively the vaccine preparations of the present invention may be used to protect or treat a mammal susceptible to, or suffering from disease, by means of administering said vaccine via a mucosal route, such as the oral/alimentary or nasal route. Alternative mucosal routes are intravaginal and intra-rectal. The preferred mucosal route of administration is via the nasal route, termed intranasal vaccination. Methods of intranasal vaccination are well known in the art, including the administration of a droplet, spray, or dry powdered form of the vaccine into the nasopharynx of the individual to be immunised. Nebulised or aerosolised vaccine formulations also form part of this invention. Enteric formulations such as gastro resistant capsules and granules for oral administration, suppositories for rectal or vaginal administration also form part of this invention.

The adjuvant combinations, represent a class of mucosal adjuvants suitable for application in humans to replace systemic vaccination by mucosal vaccination. In a preferred form pure saponins such as Quil A, or derivatives thereof, including QS21; Escin; Digitonin; or *Gypsophila* or *Chenopodium quinoa* saponins in combination with immunostimulatory oligonucleotides may be used as adjuvants for the mucosal administration of antigens to achieve a systemic immune response.

The adjuvant combinations are used in the formulation of vaccines, which vaccines may be administered via the systemic or mucosal route. Preferably, when the vaccines are used for mucosal administration the adjuvant combination comprises a haemolytic saponin.

For mucosal administration preferably the composition of the invention comprise a haemolytic saponin. Haemolytic saponin, or saponin preparation, within the meaning of this invention is to be determined with reference to the following assay.
1. Fresh blood from guinea pigs is washed with phosphate buffered saline (PBS) 3 times in a desk-top centrifuge. After resuspension to the original volume the blood is further diluted 10 fold in PBS.
2. 50 µl of this blood suspension is added to 800 µl of PBS containing two-fold dilutions of surfactant or saponin.
3. After 8 hours the haemolysis is assessed visually or by measuring the optical density of the supernatant. The presence of a red supernatant, which absorbs light at 570 nm indicates the presence of haemolysis.
4. The results are expressed as the concentration of the first saponin dilution at which hemolysis no longer occurs.

For the purposes of this invention the saponin adjuvant preparation is haemolytic if it lyses the erythrocytes at a concentration of less than 0.1%. As means of reference, substantially pure samples of QuilA, QS21, QS7, Digitonin, and β-escin are all haemolytic saponins as defined in this assay. Within the inherent experimental variability of such a biological assay, the saponins preferably have a haemolytic activity, of approximately between 0.5-0.00001 %, more preferably between 0.05-0.00001%, even more preferably between 0.005-0.00001%, and most preferably between 0.001-0.0004%. Ideally, said saponins should have a haemolytic activity similar (*i.e.* within a ten-fold difference) to that of QS21.

The vaccines of the present invention may also be administered via the oral route. In such cases the pharmaceutically acceptable excipient may also include alkaline buffers, or enteric capsules or microgranules. The vaccines of the present invention may also be administered by the vaginal route. In such cases, the pharmaceutically acceptable excipients may also include emulsifiers, polymers such as CARBOPOL^{®}, and other known stabilisers of vaginal creams and suppositories. The vaccines of the present invention may also be administered by the rectal route. In such cases the excipients may also include waxes and polymers known in the art for forming rectal suppositories.

Preparations of more than one saponin in the adjuvant combinations also form part of the present invention. For example, combinations of at least two of the following group comprising QS21, QS7, Quil A, β-escin, or digitonin. Additionally, the compositions of the present invention may comprise combinations of more than one immunostimulatory oligonucleotide.

Alternatively the formulations may be combined with vaccine vehicles composed of chitosan or other polycationic polymers, polylactide and polylactide-co-glycolide particles, poly-N-acetyl glucosamine-based polymer matrix, particles composed of polysaccharides or chemically modified polysaccharides, liposomes and lipid-based particles, particles composed of glycerol monoesters, etc. The saponins may also be formulated in the presence of cholesterol to form particulate structures such as liposomes or ISCOMs. Furthermore, the saponins may be formulated together with a polyoxyethylene ether or ester, in either a non-particulate solution or suspension, or in a particulate structure such as a paucilamelar liposome or ISCOM. The saponins may also be formulated with excipients such as Carbopol^{R} to increase viscosity, or may be formulated in a dry powder form with a powder excipient such as lactose.

Particularly preferred adjuvants are combinations of 3D-MPL and QS21 (EP 0 671 948 B1), oil in water emulsions comprising 3D-MPL and QS21 (WO 95/17210, WO 98/56414) in combination with the CpG oligonucleotides as herein described. The amount of CpG or immunostimulatory oligonucleotides in the adjuvants or vaccines of the present invention is generally small, but depending on the vaccine formulation may be in the region of 1-1000µg per dose, preferably 1-500µg per dose, and more preferably between 1 to 100µg per dose.

The amount of saponin for use in the adjuvants may be in the region of 1-1000µg per dose, preferably 1-500µg per dose, more preferably 1-250µg per dose, and most preferably between 1 to 100µg per dose. The ratio of CpG:saponin (w/w) will, therefore, be in the range of 1:1000 to 1000:1, and will typically be in the range of 1:100 to 100:1, and preferably in the range of 1:10 to 1:1 or 1:1 to 10:1, and most preferably 1:1, 4:1 or 10:1.

The formulations of the present invention maybe used for both prophylactic and therapeutic purposes. Accordingly, there is provided the use as defined in claim 6 of a combination of a saponin, a lipopolysaccharide and a CpG molecule in the manufacture of a vaccine for the prophylaxis and the treatment of cancer, in particular breast and prostate carcinomas. In a further aspect of the present invention there is provided a vaccine combination as defined in claim 7 comprising a lipopolysaccharide, a saponin and CpG, as herein described for use as a medicament. Vaccine preparation is generally described in New Trends and Developments in Vaccines, edited by Voller et al., University Park Press, Baltimore, Maryland, U.S.A. 1978.

Examples of suitable pharmaceutically acceptable excipients for use in the combinations of the present invention include water, phosphate buffered saline, isotonic buffer solutions.

None of the following examples fall within the scope of the appended claims, but illustrates the effect of the adjuvant composition

### Example 1:

- ECD-PD was produced in CHO cells according to the methods of WO 00/44899. The formulations were tested in mice and rabbits.
- Formulations were compared against a number of controls.

### SBAS1+SBAS7:

ECD-PD formulated with CpG oligonucleotide 2006 3D-MPL, QS21 in liposomes.

### SBAS1 formulation

Comprising QS21 in liposomes and 3D-MPL associated with the liposomes were prepared according to the procedures of EP 0822831.

### SBAS1+SBAS7 formulation

To the formulation above CpG oligonucleotide 2006 was added. The antigen was admixed to the adjuvant formulation prior to use.

### SBAS7 + SBAS2-based formulations ( mice)

For one dose of 50 µl of vaccine, the ECD-PD protein (25µg) was diluted in 10 fold concentrated PBS pH 6.8 and H2O before consecutive addition of an oil in water emulsion comprising SB62: which is prepared by and comprises 5% squalene 5% tocopherol 2.0% tween 80; the particle size was 180nm

### Preparation of emulsion SB62 (2 fold concentrate)

Tween 80 is dissolved in phosphate buffered saline (PBS) to give a 2% solution in the PBS. To provide 100 ml two fold concentrate emulsion 5g of DL alpha tocopherol and 5ml of squalene are vortexed to mix thoroughly. 90ml of PBS/Tween solution is added and mixed thoroughly. The resulting emulsion is then passed through a syringe and finally microfluidised by using an M11OS microfluidics machine. The resulting oil droplets have a size of approximately 180 nm., 3D-MPL (10µg), QS21 (10µg). 50µg CpG ODN 2006 were then added followed 30 minutes later by the addition of 50 µg/ml thiomersal as preservative. All incubations were carried out at room temperature with agitation.

SBAS 2 formulations were prepared as above, but without the addition of the CpG oligonucleotide.

SBAS7 is CpG oligonucleotide 2006

### SBAS7 + SBAS2-based formulations (Rabbit)

For one dose of 500/µl of vaccine, the ECD-PD protein (100µg) was diluted in 10 fold concentrated PBS pH 6.8 and H2O before consecutive addition of SB62 250µl, 3D-MPL (100µg), QS21 (100µg) and 500µg of CpG ODN 2006 followed 30 minute later, by the addition of 50 µg/ml thiomersal as preservative. All incubations were carried out at room temperature with agitation.

### Example 2: Tumour challenge experiments

Groups of F1 (C57 x Balb c) mice (8 mice/group) were injected with 1/10 of the human dose of antigen (25 µg) at days 0-14-28-42 and challenged at day 56 with TC1 cells expressing Her2 at a close 210e6 TC1 Her2 cell/animal administered subscutaneously.

TC1 cells for ½ the animals spleens were collected at day 56 and the animals bled.

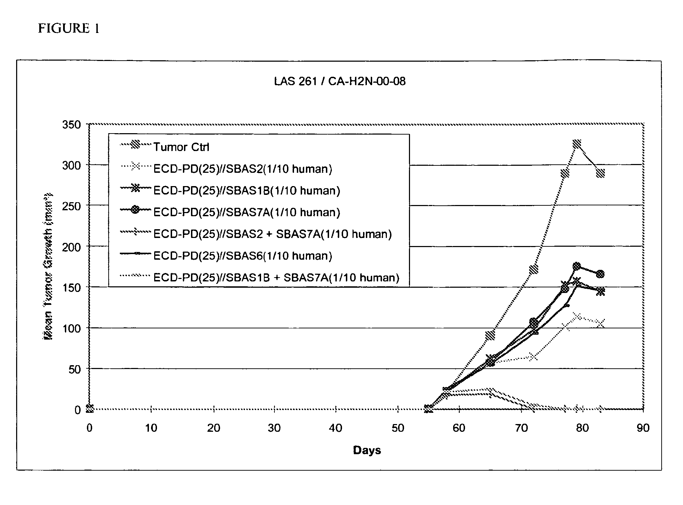

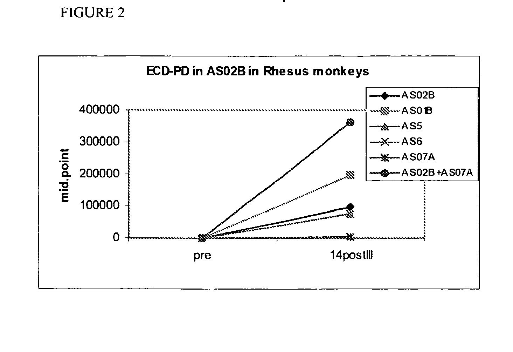

As shown in the figure 1 the addition of a CpG oligonucleotide to a 3D-MPL/QS21 formulation synergistically enhances tumour regression and only these formulations brought about complete tumour regression in the mice.

### Example 3: Immunogenicity of ECD-PD in different adjuvants in rabbits

6 groups of 4 rabbits were immunised at days 0, 21 and 42 respectively with 100µg of ECD-PD in AS02, AS01, AS05, AS06 (CpG 2006 absorbed on alum), AS07 and AS02B+AS07.

Serology was analysed 14 days post III and table 1 shows that the formulations of the present invention were superior to other formulations tested in raising high titre antibody responses.

**Table 1**

| | **pre** | **14postIII** |
|---|---|---|
| **AS02B** | 50 | 96923 |
| **AS01B** | 173 | 196637 |
| **AS5** | 144 | 76221 |
| **AS6** | 142 | 74180 |
| **AS07A** | 480 | 3904 |
| **AS02B+AS07A** | 94 | 362713 |

### Example 4: Immunogenicity of Her 2 neu, ECD-PD in adult Rhesus monkeys

Adult Rhesus monkeys were immunised with ECD-PD in various adjuvant formulations:

| | | |
|---|---|---|
| AS02 B | - | QS21, 3DMPL, in oil water emulsion |
| AS01 | - | QS21 3D-MPL in liposome |
| AS05 | - | QS21 in liposome |
| AS06 | - | CpG 2006 alum |
| AS07 | - | CpG 2006 |
| AS02B+ AS07 | - | see example 1 for details. |

Vaccination illicited a higher antibody response in the formulations of the present invention (AS)2 + AS07). See figure 1.

Further analysis showed the antibody response to be polyclonal and demonstrate an inhibitory activity on the invitro growth of a human breast cancer cell line (SKBR3) over expressing the Iter 2 neu molecule. Herceptin, a monoclonal antibody for the treatment of Her 2 neu expressing tumours is able to inhibit the growth of this cell line.

The antibodies generated after active vaccination with the formulation were thus seen to be functional.

### Example 5: Immunisation of mice with ECD-PD antigen

This experiment was designed to investigate a range of adjuvant formulations with the antigen which is a fusion of the extracellular domain of Her 2 neu linked to the phosphorylation domain (ECD-PD), which was produced in CHO cells according to the methods of WO 00/44899.

| Group | Antigen (25µg) | Adjuvant |
|---|---|---|
| 1 | ECD-PD | none (Phosphate Buffered Saline (PBS)) |
| 2 | ECD-PD | Liposomes with QS21 and 3D-MPL in membrane |
| 3 | ECD-PD | tocol containing oil in water emulsion with QS21 and 3D-MPL |
| 4 | ECD-PD | CpG |
| 5 | ECD-PD | Liposomes with QS21 and 3D-MPL in membrane + CpG |
| 6 | ECD-PD | tocol containing oil in water emulsion with QS21 and 3D-MPL + CpG |
| 7 | ECD-PD | 3D-MPL + CpG |
| 8 | ECD-PD | QS21 + CpG |
| 9 | ECD-PD | tocol containing oil in water emulsion + CpG |
| 10 | ECD-PD | Liposomes with QS21 in membrane + CpG |
| 11 | ECD-PD | Liposomes with 3D-MPL in membrane + CpG |

The tocol containing oil in water emulsions used in the above groups used D, L, -tocopherol (CAS No. 10191-41-0; chemical name: (2RS,4'RS, 8'RS)-2, 5, 7, 8-tetramethyl-2-(4',8',12'-trimethyl-tridecyl)-6-chromanol)); which is commercially available from ROCHE™. If present the tocol was present in an oil in water emulsion comprising 2.5% by volume, in combination with squalene 2.5% by volume. Both oils were mixed, and polyoxyethylene sorbitan monooleate (Tween 80™) was added, prior to microfluidisation (M110S microfluidics machine, maximum of 50 passes, for a period of 2 minutes at maximum pressure imput of 6 bar (output pressure of about 850 bar) as described in WO 95/17210). Accordingly, groups 3, 6, and 9 were based on the above tocol emulsion with the addition of aqueous QS21, 3D-MPL or CpG.

QS21 and 3D-MPL if present in any of the vaccine groups above were included at 5µg/dose; CpG (OLIGO 4 (SEQ ID NO:4): TCG TCG TTT TGT CGT TTT GTC GTT) was added at 50µg dose.

The adjuvants as used for group 2, 5, 10 were prepared according to techniques as described in EP 0 822 831 B1 (the contents of which are incorporated herein by reference). Group 11 comprised 3D-MPL in the membrane of a liposome. Briefly, the 3D-MPL, dioleoyl phosphatydyl choline and cholesterol were mixed together and microfluidised into unilamellar liposomes (as described in EP 0 822 831 B1 - with the omission of QS21).

The adjuvants used in groups 4, 7 and 8 were in aqueous suspension or solution.

### Vaccination procedure

Groups of B6F1 mice were vaccinated on four occasions (in 50µl volumes), intramuscularly, 14 days apart. 14 days post the 4th vaccine dose, the mice were challenged subcutaneously with 2X106 TC1 tumour cell expressing the Her 2 neu.

The Her 2 neu-TC1 tumour cell lines was produced by transduction of TC 1 cells by retroviral vectors coding for Her 2 neu. After a selection period with blastocydin, resistant clones were isolated and screened by FAGS for Her 2 neu expression. The clone with the highest Her 2 neu expression was selected, and a challenge dose of 2X106 was identified to have a similar Kinetic of growth as the wild-type TC1 cells and to give rise to a developing tumour in 100% of the control animals.

The size of the individual tumors were measured twice a week and expressed as a group mean.

### Results

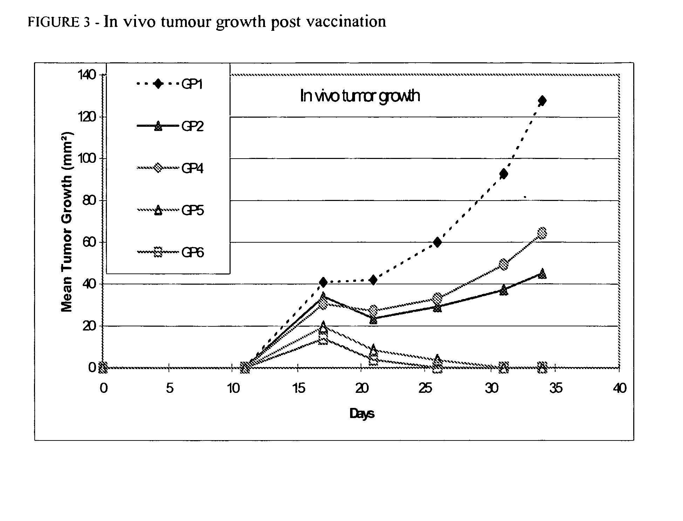

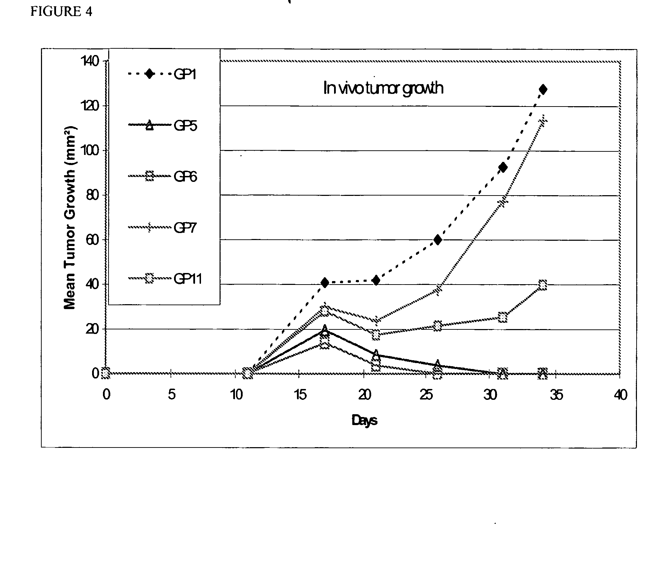

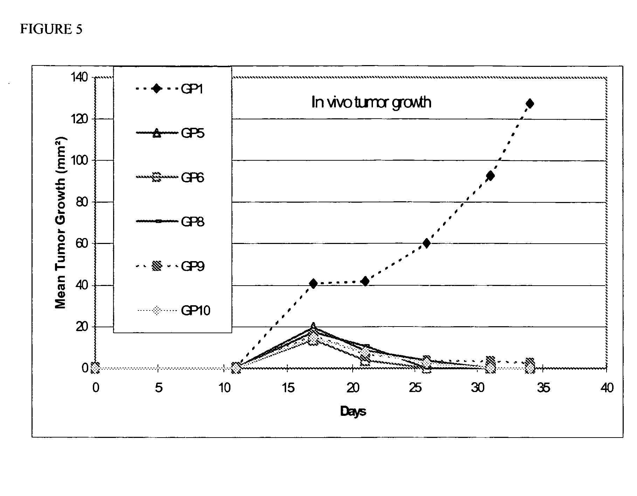

Figure 3 shows the tumour growth results for groups 1, 2, 4, 5 and 6. Figure 4 shows the tumour growth results for groups 1, 5, 6, 7 and 11. Figure 5 shows the tumour growth results for groups 1, 5, 6, 8, 9 and 10. The only vaccines that induced a complete regression of the tumour were vaccine containing both an immunostimulatory oligonucleotide and a saponin.

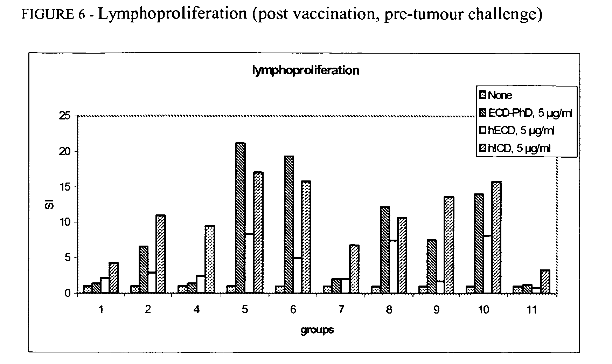

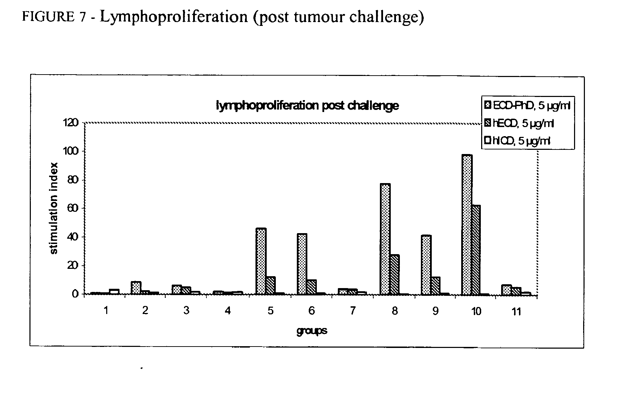

Figures 6 and 7 show the lymphoproliferation of splenocytes in vitro after incubation with the 5µg/ml of immunogen (ECD-PD) or extracellular domain (ECD) or intracellular domain (ICD) or Her 2 neu.

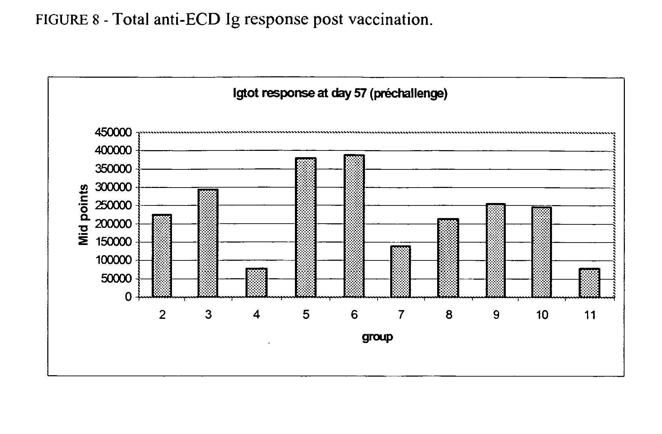

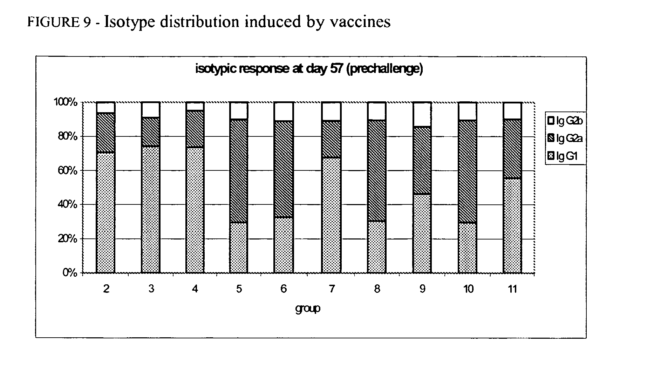

Figures 8 and 9 show the humoral immune response to the immunogen (ECD-PD) in terms of total Ig as measured by ELISA (FIG. 8) or IgG isotype distribution within these responses (FIG. 9).

### Conclusion:

Post 3 injections, the antibody induction is
AS02B+AS07A > AS01B > AS02B = AS06 = AS05 > AS07A

### General conclusion

The adjuvant tested (AS1, AS2, AS7) have similar effect. However,the combination of AS1 and AS7 or AS2 and AS7 are more effective adjuvants. CMI is clearly shown after 4 vaccinations in animals receiving the combined adjuvant on the whole molecule ECD-PD, but also on each part separately (ECD and ICD). The formulations of the present invention are very effective in inducing tumour regression.

### Example 6: Immunisation of mice with P703P antigen

This experiment was designed to investigate a range of adjuvant formulations with the antigen which is a fusion of the antigen Prostase (Ferguson, et al. (Proc. Natl. Acad. Sci. USA 1999, 96, 3114-3119)) and the N-terminal 1-81 fragment of NS1 from the Influenza virus (P703P-NS1).

| Group | Antigen (25µg) | Adjuvant |
|---|---|---|
| 1 | P703P-NS1 | none (Phosphate Buffered Saline (PBS)) |
| 2 | P703P-NS1 | CpG |
| 3 | P703P-NS1 | Liposomes with QS21 in membrane + CpG |
| 4 | P703P-NS1 | Liposomes with QS21 and 3D-MPL in membrane + CpG |
| 5 | P703P-NS1 | tocol containing oil in water emulsion with QS21 and 3D-MPL + CpG |
| 6 | P703P-NS1 | tocol containing oil in water emulsion + CpG |

The tocol containing oil in water emulsions used in the above groups used D, L, α-tocopherol (CAS No. 10191-41-0; chemical name: (2RS,4'RS, 8'RS)-2, 5, 7, 8-tetramethyl-2-(4',8', 12'-trimethyl-tridecyl)-6-chromanol)); which is commercially available from ROCHE™. If present the tocol was present in an oil in water emulsion comprising 2.5% by volume, in combination with squalene 2.5% by volume. Both oils were mixed, and polyoxyethylene sorbitan monooleate (Tween 80™) was added, prior to microfluidisation (M110S microfluidics machine, maximum of 50 passes, for a period of 2 minutes at maximum pressure input of 6 bar (output pressure of about 850 bar) as described in WO 95/17210). Accordingly, groups 5 and 6 were based on the above tocol emulsion with the addition of aqueous QS21, 3D-MPL and/or CpG.

QS21 and 3D-MPL if present in any of the vaccine groups above were included at 5µg/dose; CpG (OLIGO 4 (SEQ ID NO:4): TCG TCG TTT TGT CGT TTT GTC GTT) was added at 50µg dose.

The adjuvants as used for group 3 and 4 were prepared according to techniques as described in EP 0 822 831 B1 (the contents of which are incorporated herein by reference).

### Vaccination procedure

Groups of B6F1 mice were vaccinated on four occasions (in 50µl volumes), intramuscularly, 14 days apart.

### Results

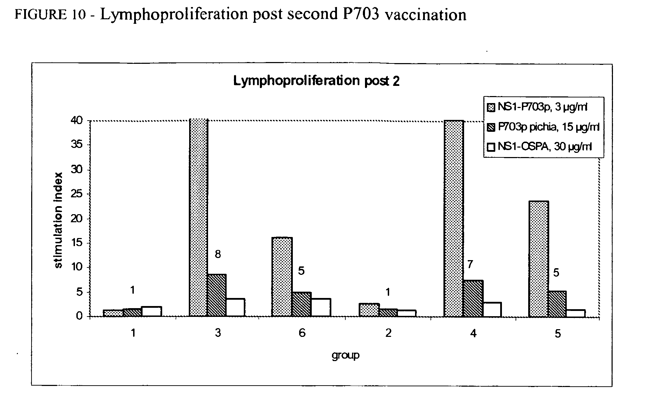

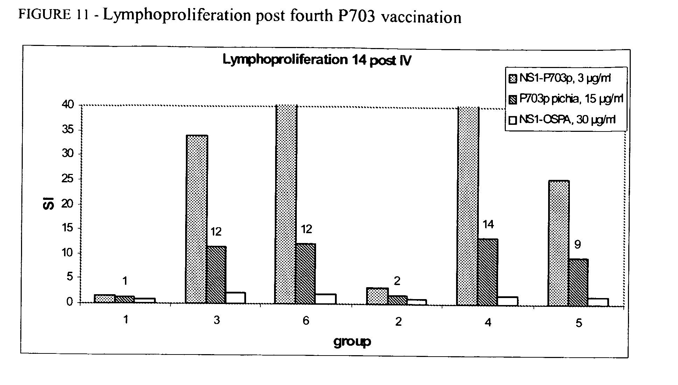

Figures 10 and 11 show the in vitro lymphoproliferation of splenocytes post second and 14 days post fourth vaccinations, after in vitro incubation with the 3µg/ml of immunogen (NS1-P703P) or pichia expressed P703P (15µg/ml) or a non-specific NS1-OspA fusion protein.

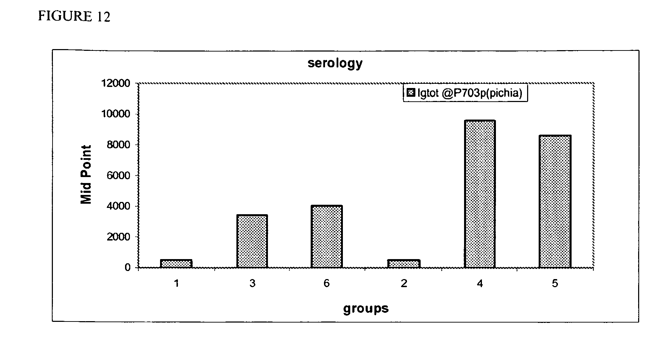

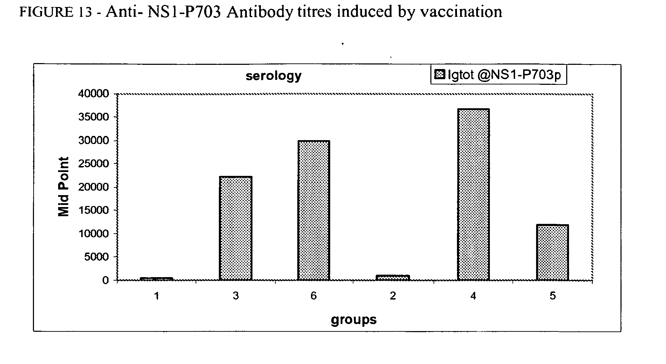

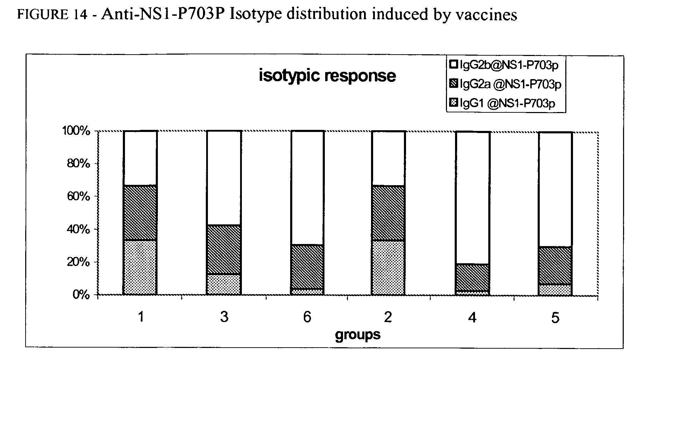

Figures 12 and 13 show the humoral immune response to the immunogen (NS1-P703P) in terms of total Ig as measured by mid-point titre ELISA (FIG. 10) or IgG isotype distribution within these responses (FIG. 11).

## Claims

1. An immunogenic composition comprising a cancer antigen selected from the group:
(i) ASCL1 (HASH-1) and
(ii) ASCL2 (PASH-2)
an adjuvant composition comprising a saponin; an immunostimulatory oligonucleotide; and a lipopolysaccharide selected from the group:
(a) Monophosphoryl Lipid A,
(b) 3-0-Deacylated Monophosphoryl Lipid A, and
(c) Disphosphoryl Lipid A.

2. A composition as claimed in claim 1 wherein the saponin is QS21.

3. An immunogenic composition as claimed in claim 1 or claim 2 wherein the immunostimulatory oligonucleotide is selected from the group:
Sequence ID No 1 - TCC ATG ACG TTC CTG ACG TT (CpG 1826)
Sequence ID No 2 - TCT CCC AGC GTG CGC CAT (CpG 1758)
Sequence ID No 3 - ACC GAT GAC GTC GCC GGT GAC GGC ACC ACG
Sequence ID No 4 - TCG TCG TTT TGT CGT TTT GTC GTT (CpG 2006)
Sequence ID No 5 - TCC ATG ACG TTC CTG ATG CT (CpG 1668)

4. A composition as claimed in any of claims 1 to 3 wherein the saponin is formulated to form ISCOMS or liposomes.

5. A composition as claimed in any of claims 1 to 3 wherein the saponin is present in an oil in water emulsion.

6. Use of a combination of a saponin; an immunostimulatory oligonucleotide; a lipopolysaccharide selected from the group:
a) Monophosphoryl Lipid A,
b) 3-0-Deacylated Monophosphoryl Lipid A, and
c) Disphosphoryl 5 Lipid A;
and a cancer antigen selected from the group:
i) (HASH-1) and
ii) (HASH-2);
in the manufacture of a medicament for the treatment or prophylaxis of tumours.

7. A combination of a saponin; an immunostimulatory oligonucleotide; a lipopolysaccharide selected from the group:
a) Monophosphoryl Lipid A,
b) 3-0-Deacylated Monophosphoryl Lipid A, and
c) Disphosphoryl Lipid A;
and a cancer antigen selected from the group:
i) ASCL1 (HASH-1), and
ii) ASCL2 (HASH-2);
for use in a method for the treatment or prophylaxis of tumours.

## Patentansprüche

1. Immunogene Zusammensetzung, umfassend ein Krebsantigen, das ausgewählt ist aus der Gruppe:
(i) ASCL1 (HASH-1) und
(ii) ASCL2 (HASH-2);
eine adjuvante Zusammensetzung, die ein Saponin umfasst; ein immunstimulatorisches Oligonukleotid; und ein Lipopolysaccharid, das ausgewählt ist aus der Gruppe:
(a) Monophosphoryl-Lipid A,
(b) 3-O-deacyliertes Monophosphoryl-Lipid A, und
(c) Diphosphoryl-Lipid A.

2. Zusammensetzung gemäß Anspruch 1, wobei das Saponin QS21 ist.

3. Immunogene Zusammensetzung gemäß Anspruch 1 oder 2, wobei das immunstimulatorische Oligonukleotid ausgewählt ist aus der Gruppe:
Sequenz-ID NO: 1 - TCC ATG ACG TTC CTG ACG TT (CpG 1826)
Sequenz-ID NO: 2 - TCT CCC AGC GTG CGC CAT (CpG 1758)
Sequenz-ID NO: 3 - ACC GAT GAC GTC GCC GGT GAC GGC ACC ACG
Sequenz-ID NO: 4 - TCG TCG TTT TGT CGT TTT GTC GTT (CpG 2006)
Sequenz-ID NO: 5 - TCC ATG ACG TTC CTG ATG CT (CpG 1668)

4. Zusammensetzung gemäß mindestens einem der Ansprüche 1 bis 3, wobei das Saponin formuliert ist, um ISCOMS oder Liposomen zu bilden.

5. Zusammensetzung gemäß mindestens einem der Ansprüche 1 bis 3, wobei das Saponin in einer Öl-in-Wasser-Emulsion vorliegt.

6. Verwendung einer Kombination aus einem Saponin; einem immunstimulatorischen Oligonukleotid; einem Lipopolysaccharid, das ausgewählt ist aus der Gruppe:
(a) Monophosphoryl-Lipid A,
(b) 3-O-deacyliertes Monophosphoryl-Lipid A, und
(c) Diphosphoryl-5-Lipid A;
und einem Krebsantigen, das ausgewählt ist aus der Gruppe:
(i) ASCL1 (HASH-1) und
(ii) ASCL2 (HASH-2);
in der Herstellung eines Medikaments zur Behandlung oder Prophylaxe von Tumoren.

7. Kombination aus einem Saponin; einem immunstimulatorischen Oligonukleotid; einem Lipopolysaccharid, das ausgewählt ist aus der Gruppe:
(a) Monophosphoryl-Lipid A,
(b) 3-O-deacyliertes Monophosphoryl-Lipid A, und
(c) Diphosphoryl-Lipid A;
und einem Krebsantigen, das ausgewählt ist aus der Gruppe:
(i) ASCL1 (HASH-1) und
(ii) ASCL2 (HASH-2);
zur Verwendung in einem Verfahren zur Behandlung oder Prophylaxe von Tumoren.

## Revendications

1. Composition immunogène comprenant un antigène cancéreux choisi dans le groupe :
(i) ASCL1 (HASH-1) et
(ii) ASCL2 (HASH-2) ;
une composition adjuvante comprenant une saponine ; un oligonucléotide immunostimulant ; et un lipopolysaccharide choisi dans le groupe :
(a) monophosphoryl-lipide-A,
(b) monophosphoryl-lipide-A 3-O-désacylé, et
(c) diphosphoryl-lipide-A.

2. Composition selon la revendication 1, dans laquelle la saponine est le QS21.

3. Composition immunogène selon la revendication 1 ou la revendication 2, dans laquelle l'oligonucléotide immunostimulant est choisi dans le groupe :
Séquence ID No. 1 - TCC ATG ACG TTC CTG ACG TT (CpG 1826)
Séquence ID No. 2 - TCT CCC AGC GTG CGC CAT (CpG 1758)
Séquence ID No. 3 - ACC GAT GAC GTC GCC GGT GAC GGC ACC ACG
Séquence ID No. 4 - TCG TCG TTT TGT CGT TTT GTC GTT (CpG 2006)
Séquence ID No. 5 - TCC ATG ACG TTC CTG ATG CT (CpG 1668)

4. Composition selon l'une quelconque des revendications 1 à 3, dans laquelle la saponine est formulée pour former des ISCOM ou des liposomes.

5. Composition selon l'une quelconque des revendications 1 à 3, dans laquelle la saponine est présente dans une émulsion huile dans l'eau.

6. Utilisation d'une combinaison d'une saponine ; d'un oligonucléotide immunostimulant ; d'un lipopolysaccharide choisi dans le groupe :
a) monophosphoryl-lipide-A,
(b) monophosphoryl-lipide-A 3-O-désacylé, et
(c) diphosphoryl-5-lipide-A ;
et d'un antigène cancéreux choisi dans le groupe :
(i) ASCL1 (HASH-1) et
(ii) ASCL2 (HASH-2) ;
dans la fabrication d'un médicament destiné au traitement ou à la prophylaxie de tumeurs.

7. Combinaison d'une saponine ; d'un oligonucléotide immunostimulant ; d'un lipopolysaccharide choisi dans le groupe :
(a) monophosphoryl-lipide-A,
(b) monophosphoryl-lipide-A 3-O-désacylé, et
(c) diphosphoryl-lipide-A ;
et d'un antigène cancéreux choisi dans le groupe :
(i) ASCL1 (HASH-1) et
(ii) ASCL2 (HASH-2) ;
pour une utilisation dans un procédé de traitement ou de prophylaxie de tumeurs.
